(19) Europäisches Patentamt
European Patent Office
Office européen des brevets

(11) Veröffentlichungsnummer: **0 290 886 B1**

(12) **EUROPÄISCHE PATENTSCHRIFT**

(45) Veröffentlichungstag der Patentschrift: **08.01.92**

(51) Int. Cl.$^{5}$: **C07C 323/22**, C07C 335/36

(21) Anmeldenummer: **88106892.8**

(22) Anmeldetag: **29.04.88**

(54) **Verfahren zur Herstellung von 2,5-Dichlorphenylthioglykolsäure.**

(30) Priorität: **09.05.87 DE 3715508**

(43) Veröffentlichungstag der Anmeldung:
**17.11.88 Patentblatt 88/46**

(45) Bekanntmachung des Hinweises auf die Patenterteilung:
**08.01.92 Patentblatt 92/02**

(84) Benannte Vertragsstaaten:
**CH DE FR GB IT LI**

(56) Entgegenhaltungen:
**GB-A- 1 183 696**

**HOUBEN-WEYL: "Methoden der organischen Chemie", Band E11, 1985, Seite 56, Georg Thieme Verlag, Stuttgart, DE**

(73) Patentinhaber: **CASSELLA Aktiengesellschaft**
**Hanauer Landstrasse 526**
**W-6000 Frankfurt am Main 61(DE)**

(72) Erfinder: **Bauer, Wolfgang, Dr. Dipl.-Chem.**
**Masurenstrasse 6**
**W-6457 Maintal 3(DE)**
Erfinder: **Steckelberg, Willi, Dr. Dipl.-Chem.**
**Goldgrabenstrasse 24**
**W-6238 Hofheim(DE)**

(74) Vertreter: **Urbach, Hans-Georg, Dr. et al**
**Hanauer Landstrasse 526**
**W-6000 Frankfurt am Main 61(DE)**

**EP 0 290 886 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues, wirtschaftlich und ökologisch vorteilhaftes Verfahren zur Herstellung von 2,5-Dichlorphenyl-thioglykolsäure der Formel IV durch Reaktion von 2,5-Dichlorphenyl-diazoniumsalzen der Formel I, worin $X^{\ominus}$ das Anion einer Mineralsäure bedeutet, mit Thioharnstoff in Gegenwart eines Katalysators zu 2,5-Dichlorphenyl-isothiuronium-Salzen der Formel II sowie anschließende Hydrolyse zu 2,5-Dichlorthiophenol der Formel III und Kondensation mit Monochloressigsäure:

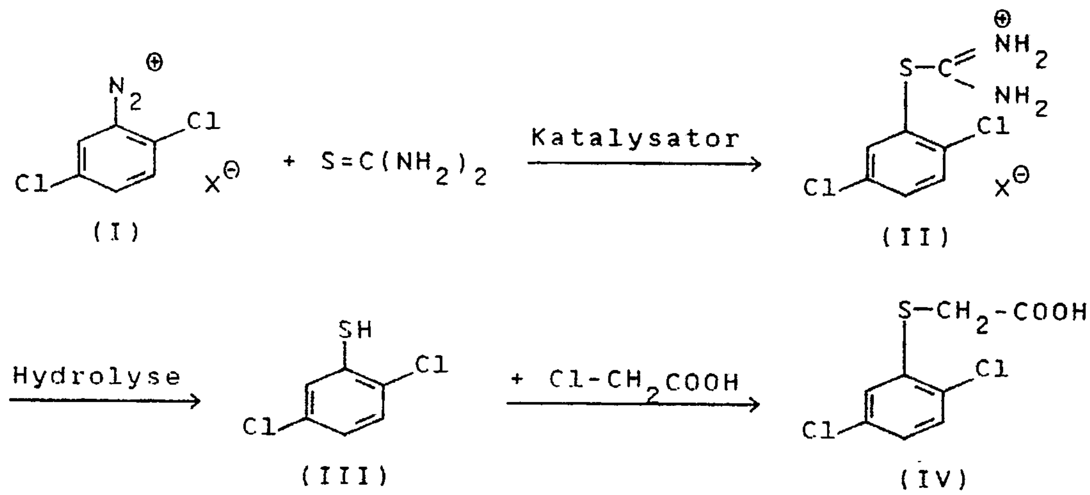


(I) (II) (III) (IV)

2,5-Dichlorphenyl-thioglykolsäure ist ein wertvolles Ausgangsprodukt zur Herstellung des Pigments Tetrachlorthioindigo.

Es sind bereits verschiedene Verfahren zur Synthese von 2,5-Dichlorphenyl-thioglykolsäure bekannt, nach denen 2,5-Dichlorphenyl-diazoniumsalze mit Thioglykolsäure umgesetzt werden.

So wird zum Beispiel in der EP-A 67 352 ein Verfahren beschrieben, wonach Aryldiazoniumsalze, beispielsweise 2,5-Dichlorphenyl-diazoniumsalze, mit Thioglykolsäure im wäßrig sauren Medium in S-Arylthioglykolsäuren, z.B. 2,5-Dichlorphenyl-thioglykolsäure übergeführt werden. Gegenüber früher beschriebenen Herstellungsverfahren von S-Arylthioglykolsäuren (DE-PS 194 040, DE-PS 201 231, DE-PS 201 232, CH-PS 451 921) bedeutet dieses Verfahren hinsichtlich der erzielbaren Ausbeute von 75 bis 84 % der Theorie zwar eine Verbesserung, erhebliche Nachteile sind jedoch durch den erforderlichen Einsatz unwirtschaftlich großer Mengen an Kupfersalzen, vorzugsweise 0,6 bis 1 Mol Kupfersalz pro Mol Aryldiazoniumsalz, gegeben.

Die resultierenden Fabrikationsabwasser weisen einen hohen Gehalt an Kupfersalzen auf, die entweder durch aufwendige, kostenintensive Aufarbeitungsmethoden entfernt werden müssen oder eine erhebliche Umweltbelastung darstellen.

Ferner müssen die zunächst anfallenden S-Arylthioglykolsäuren nach aufwendigen Methoden weiter gereinigt werden.

Nach einem weiteren, in DE-OS 3 309 142 beschriebenen Verfahren werden S-Arylthioglykolsäuren durch Umsetzung von Aryl-diazoniumsalzen mit Thioglykolsäure in einer Mischung aus Wasser und einem mit Wasser nicht oder nur teilweise mischbaren organischen Lösungsmittel, wie Chloroform, o-Dichlorbenzol, Trichlorethylen und insbesondere Perchlorethylen hergestellt.

Durch die Verwendung dieser chlorierten Kohlenwasserstoffe treten verschiedene Probleme auf, insbesondere im Zusammenhang mit kostenintensiven Methoden zur Reinigung der Abluft bei Filtrationsschritten und bei der Lösungsmittelregeneration. Ferner besteht bei dem genannten Verfahren die Gefahr der Abwasserbelastung durch die eingesetzten chlorierten Kohlenwasserstoffe.

Eine weitere Methode zur Herstellung von 2,5-Dichlorphenyl-thioglykolsäure besteht in der Sulfochlorierung von 1,4-Dichlorbenzol mit Chlorsulfonsäure und Thionylchlorid zu 2,5-Dichlorbenzolsulfochlorid, anschließender Reduktion von 2,5-Dichlorbenzolsulfochlorid mit einem großen Überschuß an Zinkpulver im schwefelsauren Medium (s.z.B. britische Patentschrift 1 183 696; Monatshefte für Chemie 48, 627 (1927); Organic Syntheses, Coll.Vol.I, 504 (1964)) zu 2,5-Dichlorthiophenol, das in einem Folgeschritt mit Monochloressigsäure zu 2,5-Dichlorphenyl-thioglykolsäure kondensiert wird (brit. Patentschrift 1 183 696).

Auch diesem Verfahren haften aus ökologischer und wirtschaftlicher Sicht eine Reihe von Nachteilen an, die vor allem durch die hohe Abwasserbelastung durch Dünnsäure bei der Isolierung von 2,5-Dichlorbenzolsulfochlorid und im Reduktionsschritt durch erhebliche Mengen an Schwermetallsalzen bei der Zinkstaubreduktion gegeben sind.

Ferner verursacht der Umgang mit feinteiligem Zinkstaub in Gegenwart von bei der Zinkstaubreduktion entstehendem nascierendem Wasserstoff sicherheitstechnische Probleme.

Der vorliegenden Erfindung lag demnach die Aufgabe zugrunde, ein umweltfreundliches Verfahren zur Herstellung von 2,5-Dichlorphenyl-thioglykolsäure bereitzustellen, das die wirtschaftlichen und ökologischen Probleme der bereits bekannten Verfahren nicht aufweist.

Überraschenderweise gelingt die Lösung dieser Aufgabe dadurch, daß man 2,5-Dichlorphenyl-diazoniumsalze der Formel I mit Thioharnstoff im wäßrigen Medium bei sauren pH-Werten in Gegenwart eines Katalysators in 2,5-Dichlorphenyl-isothiuroniumsalze der Formel II überführt, diese ohne Zwischenisolierung bei alkalischen pH-Werten zu 2,5-Dichlorthiophenol der Formel III hydrolisiert und dieses mit Monochloressigsäure in an sich bekannter Weise zu 2,5-Dichlorphenyl-thioglykolsäure der Formel IV kondensiert.

Die Umsetzung der 2,5-Dichlorphenyl-diazoniumsalze der Formel I mit Thioharnstoff wird in wäßrigem Medium bei sauren pH-Werten von 0 bis 6,5, bevorzugt von 0 bis 4, ausgeführt. Die Reaktionstemperaturen liegen in der Regel zwischen -10 und +50 °C, vorzugsweise zwischen 0 und 30 °C. Pro Mol 2,5-Dichlorphenyl-diazoniumsalz der Formel I werden im allgemeinen 1 bis 1,4 Mol, vorzugsweise 1 bis 1,25 Mol, Thioharnstoff, sowie als Katalysator 0,001 bis 0,05 Mol, vorzugsweise 0,01 bis 0,03 Mol Kupfer(I)- oder Kupfer(II)-Salz eingesetzt.

Die Hydrolyse der 2,5-Dichlorphenyl-isothiuroniumsalze der Formel II wird in der Regel bei Temperaturen zwischen 30 und 130 °C, vorzugsweise 60 und 100 °C, ausfgeführt. Das dabei entstehende 2,5-Dichlorthiophenol der Formel III kann anschließend, z.B. durch Wasserdampfdestillation oder Extraktion, zwischenisoliert werden.

In einer bevorzugten Ausführungsform des erfindungsgemäßen Verfahrens wird 2,5-Dichlorthiophenol ohne Zwishenisolierung durch Chloressigsäure-Kondensation in das Endprodukt 2,5-Dichlorphenyl-thioglykolsäure überführt.

Eine besonders bevorzugte Ausführungsform des erfindungsgemäßen Verfahrens besteht darin, daß man dem Katalysator feinteilige, nichtmetallische Festsubstanzen, die unter den Reaktionsbedingungen unlöslich und inert sind, zusetzt.

Hierdurch wird die Ausbeute an 2,5-Dichlorphenyl-thioglykolsäure noch weiter gesteigert, und die Bildung unerwünschter Nebenprodukte reduziert.

Als feinteilige, nichtmetallische Festsubstanzen sind alle wasserunlöslichen synthetischen oder natürlichen Stoffe, die als Adsorptionsmittel bei technischen Adsorptionsverfahren oder bei der Adsorptionschromatographie eingesetzt werden, geeignet, so z.B. Aktivkohlen, Tier- und Knochenkohlen, Silicate und Aluminiumsilicate, wie z.B. Siliciumdioxid (Kieselgel), Kieselgur, Bleicherden, Bentonite, Montmorrilonit, Aluminiumoxide, Bauxite, Talk, Glaspulver, Molekularsiebe. Ferner sind z.B. geeignet in Wasser unlösliche Oxide und Salze, z.B. Carbonate, Silicate oder Sulfate von Erdalkalimetallen.

Derartige nach dem erfindungsgemäßen Verfahren eingesetzte feinteilige Stoffe weisen eine spezifische Oberfläche von größer als 0.06 $m^2/cm^3$ auf.

In einem 1-molaren Ansatz werden sie in der Regel in Mengen von 1 bis 20 g, vorzugsweise 5 bis 15 g, eingesetzt. Sie können allein oder in Mischung untereinander verwendet werden.

Eine ganz besonders bevorzugte Ausführungsform des erfindungsgemäßen Verfahrens besteht darin, als Katalysator katalytische Mengen an Kupfer(I)- oder Kupfer(II)-Salzen, denen die genannten feinteiligen Stoffe zugesetzt sind, einzusetzen.

Die in katalytischen Mengen eingesetzten Kupfersalze können durch Zusatz äquimolarer Mengen an Natriumsulfid bzw. Natriumhydrogensulfid als Kupfersulfid gefällt und durch einen Filtrationsschritt von der Isolierung der 2,5-Dichlorphenyl-thioglykolsäure entfernt werden, wobei von Schwermetallsalzen freie Fabrikationsabwässer entstehen.

Die Ausbeute an 2,5-Dichlorphenyl-thioglykolsäure liegt bei dem erfindungsgemäßen Verfahren bei 83 bis 90 % der Theorie, bezogen auf 2,5-Dichlorphenyl-diazoniumsalze, was in Anbetracht des mehrstufigen Verfahrens überraschend ist, vor allem deshalb, weil in der Literatur beschrieben ist (K.-D. Gundermann und K. Hümke, in Houben-Weyl, Methoden der Organischen Chemie, Bd. E 11, S.56 (1985)), daß aus chlorsubstituierten Aryldiazoniumsalzen und Thioharnstoff und nachfolgender Hydrolyse der intermediär gebildeten Aryl-isothiuroniumchloride die entsprechend chlorsubstituierten Thiophenole nur in einer Ausbeute von 22 bis 50 % der Theorie entstehen.

Beispiel 1:

162 g 2,5-Dichloranilin werden in eine Mischung von 500 ml Wasser und 350 ml 10 N Salzsäure

eingetragen und in üblicher Weise bei 0 bis 5 °C mit einer Lösung von 76 g Natriumnitrit in 150 ml Wasser diazotiert. Die erhaltene Lösung von 2,5-Dichlorphenyl-diazoniumchlorid läuft anschließend bei einer Temperatur von 10 bis 15 °C zu einem gut gerührten Gemisch von 91,2 g Thioharnstoff, 5 g Kupfer(II)sulfat-pentahydrat und 5 g Corlite® in 500 ml Wasser. Man rührt ca. 1 Stunde nach, erhöhte den pH-Wert mit 330 ml 10 N Natronlauge auf 8,5 und hydrolysiert 1 Stunde bei 90 °C. Danach wird mit 10 N Salzsäure ein pH-Wert von 3 eingestellt und entstandenes 2,5-Dichlorthiophenol durch Wasserdampfdestillation isoliert. (Corlite® ist ein eingetragenes Warenzeichen der Corneco-Chemie GmbH., Dortmund)
Ausbeute: 154 g 2,5-Dichlorthiophenol (86 % der Theorie), Kp.: 126 bis 129 °C/10 mbar.

154 g 2,5-Dichlorthiophenol werden in 1,8 l Wasser eingetragen, mit 200 ml 10 N Natronlauge und darauf bei 80 °C mit 104 g Monochloressigsäure versetzt. Anschließend wird das Produkt bei 40 bis 50 °C mit 124 ml 10 N Salzsäure gefällt, durch Filtration isoliert, mit Wasser säurefrei gewaschen und bei 80 °C getrocknet.

Ausbeute: 194,4 g 2,5-Dichlorphenyl-thioglykolsäure (82 % der Theorie, bezogen auf 2,5-Dichloranilin); Fp.: 130 bis 132 °C.

Beispiel 2

Eine gemäß Beispiel 1 aus 162 g 2,5-Dichloranilin hergestellte 2,5-Dichlorphenyl-diazoniumchlorid-Lösung wird bei 10 bis 15 °C zu einer Mischung von 81,2 g Thioharnstoff, 5 g Kupfer(II)sulfat-pentahydrat, 5 g Aktivkohle und 10 g Corlite® in 500 ml Wasser gegeben.

Nach beendeter Stickstoffentwicklung stellt man durch Zugabe von 330 ml 10 N Natronlauge einem pH-Wert von 8,5 ein, heizt 1 Stunde auf 90 °C und gibt anschließend 200 ml 10 N Natronlauge, 800 ml Wasser und 128,8 g Monochloressigsäure zu. Man heizt auf 100 °C, versetzt mit 2,6 g Natriumsulfid 60%ig und filtriert. Die Produktisolierung erfolgt aus dem alkalischen Filtrat durch Zugabe von 10 N Salzsäure bis zu einem pH-Wert von 2, Filtration bei 20 °C, Neutralwaschen und Trocknung bei 80 °C.

Ausbeute: 211 g 2,5-Dichlorphenyl-thioglykolsäure (89 % der Theorie, bezogen auf 2,5-Dichloranilin); Fp.: 129 bis 131 °C.

Das resultierende Filtrat weist einen Kupfergehalt von < 1 ppm auf.

Beispiel 3:

Umsetzung gemäß Beispiel 2, jedoch ohne Verwendung von Aktivkohle und Corlite®.

Ausbeute: 197 g 2,5-Dichlorphenyl-thioglykolsäure (83 % der Theorie, bezogen auf 2,5-Dichloranilin); Fp: 126 bis 129 °C.

Der folgenden Tabelle sind weitere Beispiele zur Herstellung von 2,5-Dichlorphenyl-thioglykolsäure nach dem erfindungsgemäßen Verfahren zu entnehmen. Hierbei erfolgt die Durchführung nach der in Beispiel 2 wiedergegebenen Arbeitsweise, jedoch unter Verwendung der in Spalte 2 angeführten Katalysatoren. Die angegebenen Mengen beziehen sich auf 1 Mol 2,5-Dichloranilin.

| Beispiel | Katalysator | 2,5-Dichlorphenylthioglykolsäure | |
|---|---|---|---|
| | | Ausbeute (% d.Th.) | Schmelzpunkt (°C) |
| 4 | 2,7 g Kupfer(II)chlorid<br>15 g Corlite® | 86 | 128 bis<br>130 |
| 5 | 2,5 g Kupfer(I)chlorid<br>7,5 Aktivkohle | 85 | 127 bis<br>130 |
| 6 | 5 g Kupfer(II)sulfatpentahydrat<br>2,5 g Aktivkohle<br>2,5 g Aluminiumoxid | 84 | 127 bis<br>130 |
| 7 | 5 g Kupfer(II)sulfatpentahydrat<br>4 g Aktivkohle<br>8 g Silicagel | 88 | 128 bis<br>131 |

**Patentansprüche**

1. Verfahren zur Herstellung von 2,5-Dichlorphenyl-thioglykolsäure der Formel IV

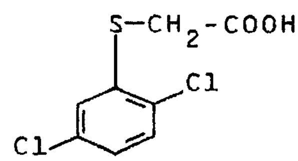

(IV)

dadurch gekennzeichnet, daß 2,5-Dichlorphenyl-diazoniumsalze der Formel I

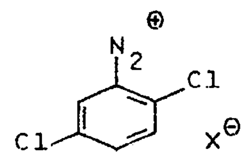

(I)

worin $X^{\ominus}$ das Anion einer Mineralsäure bedeutet, mit Thioharnstoff in wäßrigem Medium bei sauren pH-Werten in Gegenwart eines Katalysators zu 2,5-Dichlorphenyl-isothiuroniumsalzen der Formel II

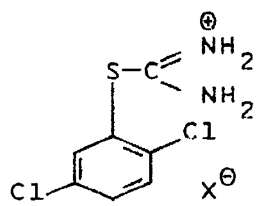

(II)

worin $X^{\ominus}$ die oben angegebene Bedeutung besitzt, umgesetzt werden, diese ohne Zwischenisolierung bei alkalischen pH-Werten zu 2,5-Dichlorthiophenol der Formel III

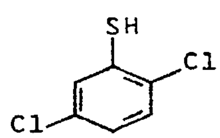

(III)

hydrolisiert werden und dieses mit Monochloressigsäure in an sich bekannter Weise zum Endprodukt kondensiert wird.

2. Verfahren gemäß Anspruch 1, dadurch gekennzeichnet, daß dem Katalysator feinteilige, nichtmetallische Festsubstanzen zugesetzt werden.

3. Verfahren gemäß Ansprüchen 1 und 2, dadurch gekennzeichnet, daß als Katalysator Kupfer(I)- oder Kupfer (II)-salze eingesetzt werden.

4. Verfahren gemäß Ansprüchen 1 bis 3, dadurch gekennzeichnet, daß als Katalysator ein Gemisch von Kupfer(I)-oder (II)-Salzen und feinteiligen, nichtmetallischen Festsubstanzen eingesetzt wird.

5. Verfahren gemäß Ansprüchen 1 bis 4, dadurch gekennzeichnet, daß pro Mol 2,5-Dichlorphenyl-diazoniumsalz 1 bis 1,4 Mol, bevorzugt 1 bis 1,25 Mol, Thioharnstoff eingesetzt wird.

6. Verfahren gemäß Anspruch 4, dadurch gekennzeichnet, daß pro Mol 2,5-Dichlorphenyl-diazoniumsalz 0,001 bis 0,05 Mol, bevorzugt 0,01 bis 0,03 Mol, Kupfer(I)- oder Kupfer(II)-Salz und 1 bis 20 g, bevorzugt 5 bis 15 g, einer feinteiligen, nichtmetallischen Festsubstanz eingesetzt werden.

7. Verfahren gemäß Ansprüchen 1 bis 6, dadurch gekennzeichnet, daß 2,5-Dichlorthiophenol zwischenisoliert wird.

8. Verfahren gemäß Ansprüchen 1 bis 6, dadurch gekennzeichnet, daß 2,5-Dichlorthiophenol ohne Zwischenisolierung in einem Eintopfverfahren mit Monochloressigsäure umgesetzt wird.

## Claims

1. Process for the preparing 2,5-dichlorophenyl-thioglycolic acid of the formula IV

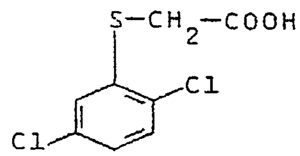

(IV)

characterized in that 2,5-dichlorophenyl-diazonium salts of the formula I

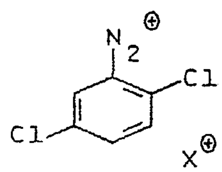

(I)

wherein $X^{\ominus}$ is the anion of a mineral acid, are reacted with thiourea in an aqueous medium at acidic pH values in the presence of a catalyst to give 2,5-dichlorophenylisothiuronium salts of the formula II

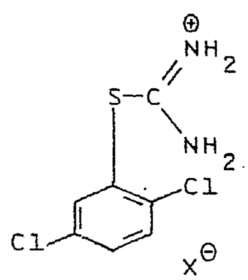

(II)

wherein $X^{\ominus}$ is as defined above, these are hydrolysed, without intermediate isolation, at alkaline pH values to give 2,5-dichlorothiophenol of the formula III

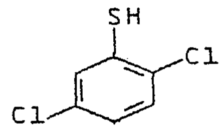

(III)

and the latter is condensed with monochloroacetic acid in a manner known per se to give the end product.

2. Process according to Claim 1, characterised in that finely disperse, non-metallic solid substances are added to the catalyst.

3. Process according to Claims 1 and 2, characterized in that copper(I) salts or copper(II) salts are used as the catalyst.

4. Process according to Claims 1 to 3, characterized in that a mixture of copper(I) or (II) salts and finely disperse, non-metallic solid substances is used as the catalyst.

5. Process according to Claims 1 to 4, characterized in that 1 to 1.4 mol, preferably 1 to 1.25 mol, of thiourea are employed per mol of 2,5-dichlorophenyl-diazonium salt.

6. Process according to Claim 4, characterized in that 0.001 to 0.05 mol, preferably 0.01 to 0.03 mol, of copper(I) salt or copper(II) salt and 1 to 20 g, preferably 5 to 15 g, of a finely disperse, non-metallic solid substance are employed per mol of 2,5-dichlorophenyldiazonium salt.

7. Process according to Claims 1 to 6, characterized in that 2,5-dichlorothiophenol is isolated as an intermediate.

8. Process according to Claims 1 to 6, characterized in that 2,5-dichlorothiophenol is reacted, without intermediate isolation, with monochloroacetic acid in a one-pot process.

## Revendications

1. Procédé de préparation de l'acide 2,5-dichlorophényl-thioglycolique de formule IV :

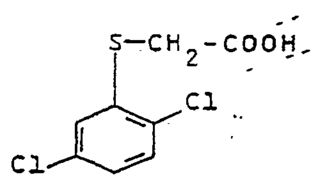

(IV) ,

caractérisé en ce qu'on fait réagir des sels de 2,5-dichlorophényl-diazonium de formule I :

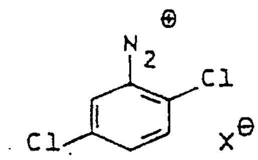

(I) ,

dans laquelle $X^-$ représente l'anion d'un acide minéral, avec de la thio-urée en milieu aqueux à des valeurs de pH acides en présence d'an catalyseur pour donner des sels de 2,5-dichlorophényl-isothiouronium de formule II :

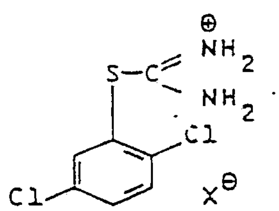

(II) ,

dans laquelle $X^-$ possède la signification indiquée plus haut, on hydrolyse ceux-ci sans isolement intermédiaire à des valeurs de pH alcalines en 2,5-dichloro-thiophénol de formule III :

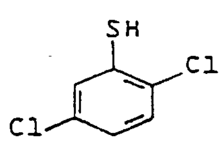

(III) ,

et on condense celui-ci en le produit final avec de l'acide monochloroacétique, de manière en soi connue.

2. Procédé selon la revendication 1, caractérisé en ce qu'on ajoute au catalyseur des substances solides non métalliques, finement divisées.

3. Procédé selon les revendications 1 et 2, caractérisé en ce qu'on utilise comme catalyseur des sels de cuivre(I) ou de cuivre(II).

4. Procédé selon les revendications 1 à 3, caractérisé en ce qu'on utilise comme catalyseur un mélange de sels de cuivre(I) ou (II) et des substances solides non métalliques, finement divisées.

5. Procédé selon les revendications 1 à 4, caractérisé en ce qu'on met en oeuvre par mol de sel de 2,5-dichlorophényl-diazonium, 1 à 1,4 mol, de préférence 1 à 1,25 mol de thio-urée.

6. Procédé selon la revendication 4, caractérisé en ce qu'on met en oeuvre par mol de sel de 2,5-dichlorophényl-diazonium, 0,001 à 0,05 mol, de préférence 0,01 à 0,03 mol de sel de cuivre(I) ou de cuivre (II) et 1 à 20 g, de préférence 5 à 15 g d'une substance solide non métallique, finement divisée.

7. Procédé selon les revendications 1 à 6, caractérisé en ce qu'on isole le 2,5-dichlorothiophénol de façon intermédiaire.

8. Procédé selon les revendications 1 à 6, caractérisé en ce qu'on fait réagir le 2,5-dichlorothiophénol avec l'acide monochloroacétique, sans isolement intermédiaire, dans un procédé en un seul pot.